# EUROPEAN PATENT APPLICATION

(11) **EP 2 610 326 A1**
(43) Date of publication of application: **03.07.2013**
(21) Application number: 10856337.0
(22) Date of filing: 24.08.2010
(51) Int. Cl.: C10J 3/16, F03G 6/00, F24J 2/00

(54) **INDUSTRIAL METHOD FOR OBTAINING LOWER ALCOHOLS FROM SOLAR POWER**

(30) Priority: 24.08.2010 ES 201031280 U
(71) Applicant: Guradoor, S.L., 38390 Santa Úrsula (Tenerife) (ES)
(72) Inventor: GONZÁLEZ GONZÁLEZ, Daniel, E-38390 Santa Úrsula (Tenerife) (ES)
(74) Representative: Gil-Vega, Victor
(86) International application number: PCT/ES2010/070568
(87) International publication number: WO 2012/025642

(57) **Abstract**

The procedure according to the invention allows the obtaining of lower alcohols from the solar energy produced at a high temperature solar thermal power plant which provides, from an energy point of view, the power supply necessary for every step of the procedure, supplying both the electricity power necessary to perform the intermediate steps of the procedure and, essentially, the products involved in the different steps (H₂, O₂, steam and CO₂) starting from a supply of wet milled coal, wherein the by-products obtained during these different stages of the procedure are fed back to the procedure itself. The procedure allows the storage of the energy obtained from the sun as lower alcohols, and such alcohols, in turn, may become an alternative to the consumption of fossil fuels, eliminating the risk derived from the production of residues; consequently, it is an especially advantageous procedure, both from an environmental and a production point of view.

## Description

The present invention refers to an industrial procedure for the obtaining of lower alcohols, preferably with less than three carbon atoms, and especially methanol and ethanol, from the energy obtained from a high temperature solar thermal power plant, using coal and water vapour as raw materials.

Specifically, the procedure disclosed in this invention allows the obtaining of lower alcohols in a manner in which the solar power obtained from a high temperature solar thermal power plant provides, from an energy point of view, the power supply necessary for every step of the procedure, supplying both the electricity power required to perform the intermediate steps of the procedure and, essentially, the products involved in the different steps (H₂, O₂, steam and CO₂) starting from a supply of wet milled coal, wherein the by-products obtained during these different stages of the procedure are fed back to the procedure itself. Consequently, this is a procedure which, on the one hand, allows to store as lower alcohols the energy obtained from the sun, and such alcohols, in turn, may become an alternative to the consumption of fossil fuels which, in turn, may be transformed into industrial fuels and/or fuels for home use and, on the other hand, as a result of the reuse of the by-products obtained during the different stages of the procedure, the procedure eliminates the risk derived from the production of residues and, consequently, it is a especially advantageous procedure, both from an environmental and a production point of view.

Currently, the entire methanol produced worldwide is synthesised from carbon monoxide and hydrogen through a catalytic process, a reaction which calls for high temperatures and pressures, as well as large and complex industrial reactors. Basically, methanol is obtained from the so-called syngas, consisting of CO, CO₂ and H₂,

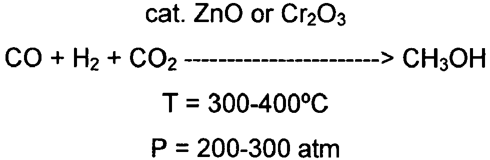

This synthesis gas can be obtained in different ways, and the process which currently is the most commonly used to obtain it is the partial combustion of natural gas in the presence of water vapour, or the partial combustion of mixtures of liquid hydrocarbons or coal in the presence of water.

Starting from this synthesis gas, the industrial processes implemented to obtain methanol are widely known, and the ones most commonly applied are those developed by the companies Lurgi Corp. and Imperial Chemical Industries Ltd. (ICI).

In short, the so-called Lurgi process, also called low-pressure process, which is used to obtain methanol from gaseous and liquid hydrocarbons or coal is based in the following steps:
1. Reforming:
   This is the step that differentiates the various processes, on the basis of the type of supply. If the supply consists of natural gas, this latter is desulphurized before feeding the reactor. Approximately half of the supply enters the first reactor, which is fed with medium pressure water vapour.
   The partial oxidation of natural gas occurs inside the reactor. This way it can be obtained H₂, CO, CO₂ and 20% of residual CH₄. The reaction takes place at 780°C and 40 atm. The syngas plus the residual methane obtained from the first reactor are mixed with the other half of the supply (previously desulphurized). The mixture of gases enters the second reactor, which is fed with the O₂ obtained from a plant used to produce oxygen from air.

      CH₄+CO+CO₂+O₂→CO+CO₂+H₂

   This reaction occurs at 950°C. In case that the fuel supplied is a liquid or coal, it is partly oxidized with O₂ and water vapour at 1,400-1,500°C and 55-60 atm. The gas thus obtained consists of H₂, CO with some impurities, consisting of small amounts of CO₂, CH₄, H₂S and free carbon. Then, the mixture passes to another reactor where the syngas is conditioned through the elimination of free carbon, H₂S and part of CO₂, and the gas remaining is ready to be fed to the methanol reactor.
2. Synthesis:
   The syngas is compressed at 70-100 atm and preheated. Then, it is fed to the methanol synthesis reactor along with the recirculation gas. The Lurgi reactor is a tubular reactor with the tubes packed with catalyst and cooled with boiling water outside. Thus, the reaction temperature is maintained between 240-270°C.

      CO + H₂ → CH₃OH ΔH < 0

      CO₂ + H₂ → CH₃OH ΔH < 0

      A significant amount of the heat generated by the reaction is transmitted to the boiling water, thus obtaining between 1 and 1,4 Kg. steam per each Kg of methanol. Furthermore, catalysts are also protected.
3. Distillation
   The gaseous methanol leaving the reactor must be purified. To that effect, it firstly passes through a heat exchanger which lowers its temperature, and the methanol is condensed. It is subsequently separated by means of a separator, from which gases are obtained, that are conditioned (appropriate temperature and pressure) and recirculated. The liquid methanol flowing from the separator is used to feed a distillation column fed with low pressure water vapour. The methanol leaves the distillation tower under standard conditions.

In the case of the ICI process, the catalytic synthesis occurs within a fluidized bed reactor, wherein the syngas is fed through the base and the methanol flows from the top. Thus, the catalyst is kept fluidized within the reactor, which is cooled with boiling water, thus obtaining steam that can be used in other stages of the process. In this process, the distillation takes place in two steps, instead of one, as it is the case with the Lurgi process.

On the other hand, the industrial production of ethanol is mainly based on the processing of biologic materials, especially certain plants which contain sugars. The ethanol thus obtained is known as bioethanol. Ethanol can also be obtained through the chemical modification of ethylene through hydration. In the first case, the bioethanol can be obtained from a certain number of plants, which involves changes in the efficiency between the fuel consumed and the fuel generated in such process, depending on the agricultural product used. This ethanol is the object of a growing controversy, since, while on the one hand it is considered as a potentially sustainable energy resource which may imply environmental and financial advantages in the long term, as opposed to fossil fuels, on the other hand it is the cause of large deforestations and of the increase in the price of food, as a result of the supplanting of forests and farming lands for its production (Monbiot, George (2008). "Calor. Cómo parar el calentamiento global", Barcelona: RBA libros, ISBN 978-84-9867-053-0), furthermore, its energy profitability has raised doubts.

The current methods of ethanol production use a significant amount of energy, when compared with the energy obtained from the fuel obtained.

Since ancient times, ethanol is obtained from the anaerobic fermentation of sugars (sucrose) with yeasts in an aqueous solution and its subsequent distillation. The process based on starch is more complex than the one based on the sucrose, since the starch must be subject to a prior hydrolysis to convert it into sugars. To that effect, the ground vegetable is mixed with water and an enzyme (or in its absence, with acid) and the pulp obtained is heated at 120 - 150°C. Subsequently, the paste is strained, a process called scarification, and sent to the fermentation reactors. The process based on cellulose is even more complex, since the vegetable stuff must be treated beforehand so that the cellulose can be subsequently attacked by the hydrolysing enzymes. The pre-treatment may consist of a combination of trituration, pyrolysis, and attack with acids and other substances. This is one of the factors that explain why the ethanol efficiency is high in the case of sugar cane, average in the case of corn and poor in the case of timber. The fermentation of sugars is carried out by microorganisms (yeasts or bacteria) and produces ethanol, as well as large amounts of CO₂. Furthermore, other oxygenated compounds are obtained, such as methanol, higher alcohols, acids and aldehydes. The fermentation typically requires some 48 hours.

The most ancient method to separate ethanol from water is simple distillation, but purity is limited to 95-96%, as a result of the formation of a water-ethanol azeotrope with a low boiling point. During the distillation process, a first fraction is obtained, which mainly contains methanol, obtained in the secondary reactions, and this is the only authorised method to obtain ethanol for human consumption. In order to obtain water-free ethanol, the azeotropic distillation is applied in a mixture with benzene or ciclohexane. The azeotrope, which comprises the ancillary solvent and water, is distilled from these mixtures at lower temperatures, while the ethanol is retained. Another purifying method, which is commonly used nowadays, consists of the physical adsorption by means of molecular sieves. At laboratory scale, desiccants like magnesium, which reacts with water to form hydrogen and magnesium oxide, can also be used. Ethanol for industrial uses can be synthesised by means of the catalytic hydration of ethylene whit sulphuric acid as catalyst. The ethylene is usually obtained from methane (one of the components of natural gas) or naphtha (an oil product). After the synthesis, a mixture of ethanol and water is obtained, which must be subsequently purified by means of any of the processes described above. According to some sources, this process is less expensive than the traditional fermentation, but currently only represents 5% of the ethanol production capacity at worldwide level.

As an alternative to the biochemical processes used for the conversion of lignocellulosic biomass into bioethanol, ABNT (Abengoa Bioenergia Nuevas Tecnologías, Abengoa S.A.) has identified the thermo-chemical processes as a potential technological route to transform the biomass into ethanol. In general terms, thermo-chemical processes are characterized in that they do not require the action of microorganisms to transform raw materials and they usually work at higher temperatures with the action of catalysts to improve chemical reactions. Thermo-chemical processes have the advantage that they may use a broad range of broad materials and in fact, any material which contains carbon may be transformed trough thermo-chemical processes; furthermore, these processes may give rise to a broad range of products, even beyond ethanol.

The thermo-chemical route is currently split into two basic stages, namely, a first stage where the biomass is transformed into an intermediate product, the syngas, and a second transformation stage, wherein the intermediate product - syngas - is transformed into the desired products.

The initial transformation of biomass into syngas is called gasification. This process is performed at very high temperatures, which typically range between 800°C and 1.400°C, and the biomass is transformed into a mixture of gases, mainly hydrogen and carbon monoxide. The syngas generated from the biomass, after the appropriate conditioning, is transformed by means of metallic catalysts that convert the hydrogen and carbon monoxide found in the syngas into a mixture of alcohols, wherein the majority product is ethanol, although other products, like higher and oxygenated alcohols, are also synthesised.

ES 2 310 127 B1, "A Procedure to obtain Syngas, a device to implement such procedure and applications" describes how to obtain a syngas using a carbonaceous material which simultaneously acts as catalyst and microwave collector, which is subject to radiation and heating by microwaves, which comprises the steps of microwave radiation and heating of the carbonaceous material, until the reaction temperature is reached, preferably between 500°C and 1000°C, and more preferably 800°C; passing of the initial gas, which comprises a mixture of CH₄ and CO₂ through the above mentioned material, maintaining the microwave radiation and heating, preferably between 500°C and 1000°C, and more preferably at 800°C; and recovery of the syngas.

ES 2 200 890 T3 relates to a procedure to synthesise methanol from hydrogen, carbon monoxide and carbon dioxide under pressure, wherein desulphurated natural gas is fed to a cracker and, subsequently, the syngas obtained from a synthesis of methanol is also fed, wherein once the syngas flow has passed through the cracker, a side stream is guided towards a methanol pre-reactor. The methanol obtained at the pre-reactor is fed with the methanol synthesis of the methanol stream deviated from the main stream, and it is fed again with a syngas stream which has not been transformed at the methanol pre-reactor, wherein, within the area of this feeding, an additional syngas is simultaneously fed, to offset the resulting loss.

ES 2 087 424 T3 relates to a gasification process implemented to obtain a syngas using solar power, which comprises: the obtaining of a liquid dispersion of a specific carbonaceous material; the provision of a solar gasification reactor with upper and lower regions adapted for the admission of a highly concentrated solar radiation; the association of such solar gasification reactor with a system of high concentration of solar radiation, adapted to obtain inside the reactor a focal elongated area with high temperatures; the continuous injection of such dispersion in such upper area of the said solar gasification reactor as droplets or discrete jets, allowing the dispersion injected in such way to sink through such elongated focal area for the effect of gravity; and the continuous evacuation of the syngas obtained from such upper region.

Considering the above mentioned background, the object of the present invention consists of providing a procedure that allows obtaining lower alcohols from the solar energy obtained from a high temperature solar thermal power plant, so that such energy can be used as the power supply source for all the steps of the procedure, providing both the power required to perform the intermediate steps of the procedure and essentially all the products required for the different stages (H₂, O₂, water vapour and CO₂), contained in the supply of wet milled coal, wherein the by-products obtained in these different stages are fed to the process again. The procedure according to the invention allows the storage of solar energy as lower alcohols that, in turn, may be used as alternative fuels, instead of fossil fuels, or be transformed into industrial and/or domestic fuels. On the other hand, and as a result of the reuse of the by-products obtained during the different stages of the procedure, the risks derived from the production of residues is essentially eliminated and, consequently, this procedure is essentially advantageous from an environmental point of view, but it is also highly efficient from an energy point of view.

Thus, the procedure according to this invention is developed by adding wet milled coal to the system, as well as water vapour from a high temperature solar thermal power plant, through a series of different steps that will be specified hereinafter, to obtain with the appropriate energetic and chemical balance, and through a series of different catalytic synthesis reactions with the relevant catalysts, the final products as lower alcohols, preferably of less than three carbon atoms which, optionally, can be transformed into fuels through a series of adequate procedures that are well known in the art.

Basically, the procedure according to the invention is based on the obtaining of a syngas from the external feeding of wet milled coal and the solar energy obtained from a high temperature solar thermal power plant, which is obtained as water vapour. In this case, the water vapour thus obtained is used both per se, as a reagent in the relevant catalytic reactors, for the development of the chemical reactions involved in the procedure, and for the generation of electric power by means of an engine/turbine device and another one of the dynamo/alternator type. This electric power allows performing an electrolysis reaction inside an electrolytic tank to obtain oxygen, which is a product necessary to produce syngas, and hydrogen, which is a reagent used for the production of lower alcohols inside the relevant catalytic reactors.

Preferably, the procedure according to the invention uses the power and the water vapour obtained from the high temperature solar thermal power plant described in patent ES2274693, which was filed by the applicant, in order to obtain a flow of water vapour, as well as an adequate pressure and temperature.

The following figure has been attached to this description, for illustration purposes and without limitation, to provide a better understanding of the procedure according to the invention:

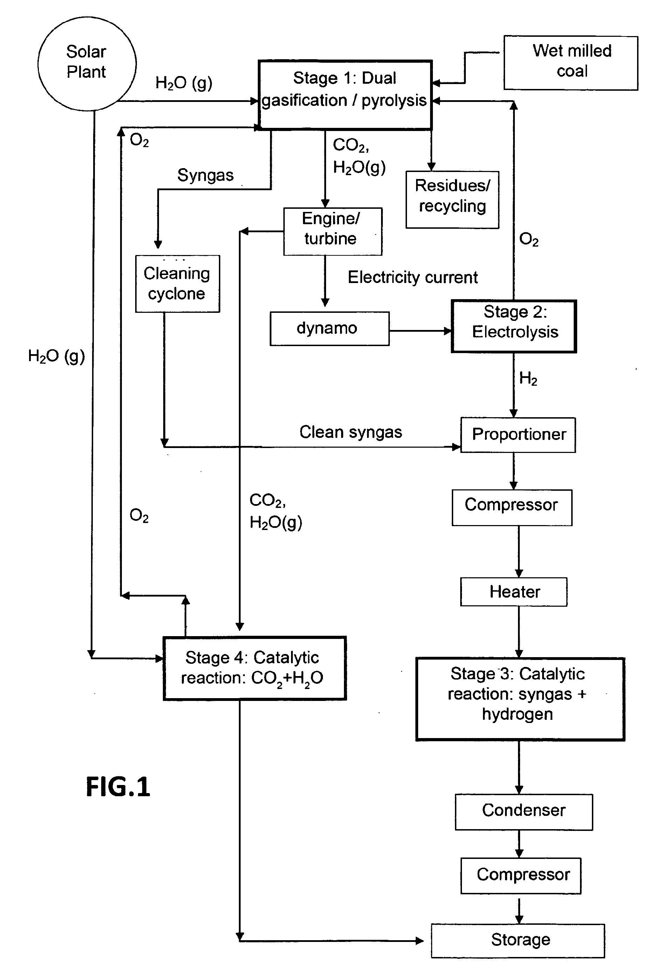

Figure 1: Shows a general diagram of the procedure according to the invention, according to a preferred embodiment of such invention.

According to figure 1, the procedure intended for the industrial production of lower alcohols, preferably with less than three carbon atoms, and particularly methanol and ethanol, from the power and the water vapour obtained from a high temperature solar thermal power plant, by adding wet milled coal, essentially consists of the following stages:

### 1. Gasification / pyrolysis within a dual reactor

The gasification and pyrolysis reactions of the wet milled coal occur during this stage of the procedure. The water vapour obtained from a high temperature solar thermal power plant is fed to a dual gasification/ pyrolysis reactor which has been previously loaded with wet milled coal through a feeding hopper. The wet milled coal is partially oxydized with O₂ obtained from a subsequent electrolysis stage (stage 2) and water vapour (obtained from the solar power plant), at a gasifier provided inside the dual reactor. The gas thus obtained basically consists of H₂, CO, and small amounts of CO₂, CH₄, H₂S and free carbon. To eliminate such traces and to condition the syngas, a pyrolysis is implemented at the reactor itself which essentially eliminates free carbon, H₂S and part of CO₂. In brief, the de-volatilization occurs between 38 and 705°C, and the carbon and hydrogen are released at a temperature ranging between 705 and 1480°C, in the case of C and H₂O(g) and between 1480 and 1815°C in the case of C and O₂.

CH₄ + CO + CO₂ + O₂ → CO + CO₂ + H₂

Similarly, the reactor comprises the appropriate catalysts required so that the different reactions may occur, as well as the necessary means to maintain its integrity, such as refractory claddings, insulating materials, refrigeration sheaths, etc. As it is shown in figure 1, the residues obtained from this dual process are collected and stored for their subsequent reuse for other processes, for instance, those processes where ashes are employed, or for their recycling as fertilizers.

The syngas thus obtained is carried to a cyclone to remove any solid residue and, subsequently, this cleaned syngas is brought towards a new reactor (stage 3).

### 2. Parallel electrolysis

The mixture of water vapour and residual CO₂ from the first stage passes through a turbine/engine so that, by means of a dynamo (continuous current), it can be generated the power required to unleash an electrolysis reaction at the appropriate electrolytic tank, which has been previously fed with demineralised water, so that the oxygen generated feeds the dual gasification / pyrolysis reactor of the stage 1 above, and the hydrogen obtained is brought to a proportioner where, along with the clean syngas from the cyclone is compressed and heated for its subsequent reaction during the third stage. Similarly, part of the mixture is brought towards a catalytic reactor from the circuit that feeds to the turbine the gas mixture composed by carbon dioxide and water vapour so that it can be used in a subsequent stage 4.

### 3. Catalytic reaction to obtain lower alcohols from syngas and hydrogen

The syngas obtained in the stage No. 1 is compressed and heated before being fed to a catalytic reactor to obtain methanol. The reactor, for instance, can be of a Lurgi type, a tubular reactor whose tubes are filled with catalyst and their outer part has been cooled with boiling water, also from the solar thermal power plant. Thus, the reaction temperature is maintained between 240-270°C.

CO + H₂ → CH₃OH ΔH < 0

CO₂ + H₂ → CH₃OH ΔH < 0

The alcohol thus obtained is kept at a storage tank for its subsequent use; or it is used to obtain industrial and domestic fuels, for instance.

### 4. Catalytic reaction to obtain lower alcohols from CO₂ and water vapour

The CO₂ and the water vapour obtained in the stage No. 1 from the initial water vapour source are recirculated towards a catalyst reactor, where the lower alcohols are obtained, according to the following reactions:

CO₂ + H₂O → CH₃OH + O₂ (75%)

CO₂ + H₂O → C₂H₅OH + O₂ (25%)

To that effect, the appropriate catalysts are arranged in the reactor, so that the reaction occurs at a temperature of 420° and at atmospheric pressure. The oxygen which is the by-product of the catalysed reaction is then recirculated towards the dual gasification / pyrolysis reactor of the first stage. The alcohols thus obtained are stored at a storage tank for their subsequent use, or can be used to produce, for instance, industrial and domestic fuels.

## Claims

1. Industrial procedure to obtain lower alcohols from solar energy, **characterised in that** it comprises the following stages:
i) gasification / pyrolysis at a dual reactor, to obtain syngas: the water vapour from a high temperature solar thermal power plant is fed to a dual gasification / pyrolysis reactor where wet milled coal has been previously fed through a feeding hopper, and such coal is partly oxidized by the O₂ obtained from a subsequent electrolysis stage (stage 2), and by the water vapour from the solar plant at a gasifier provided inside the dual reactor; the gas thus obtained is subsequently subject to a pyrolysis, removing the free carbon, the H₂S and part of CO₂, using the appropriate catalysts to obtain syngas.
ii) Electrolysis in parallel to obtain hydrogen and oxygen: the mixture of water vapour and residual CO₂ obtained at the stage i) passes through a turbine / engine to generate, by means of a dynamo, the power required to unleash an electrolysis reaction at the appropriate electrolysis tank which has been previously fed with demineralised water, so that the oxygen obtained feeds the dual gasification / pyrolysis reactor of the stage i) above, and the hydrogen obtained is brought to a proportioner where it is compressed and heated along with the syngas for its subsequent reaction in the stage iii) below, while the residual mixture of carbon dioxide and water vapour is brought from the turbine towards a catalytic reactor, so that it can be used at a subsequent stage iv).
iii) Catalytic reaction to obtain lower alcohols from syngas and hydrogen: the syngas obtained at the stage i) compressed and heated before being fed to a catalytic reactor along with the hydrogen obtained during the stage ii), to obtain lower alcohols through the relevant catalysts.
iv) Catalytic reaction to obtain lower alcohols from CO₂ and water vapour: the CO₂ and the water vapour obtained at the stage i) from the initial source of water vapour are recirculated towards a catalyst reactor where lower alcohols are obtained through the use of the appropriate catalysts, and the oxygen obtained as a by-product is recirculated towards the dual gasification/ pyrolysis reactor of the stage i).

2. Industrial procedure to obtain lower alcohols from solar energy, according to the claim 1, **characterized in that** the syngas obtained during the stage i) is also subject to a cleaning process inside a cyclone, in order to remove any solid residue and, once it has been cleaned, this syngas is fed at the stage iii).

3. Industrial procedure to obtain lower alcohols from solar energy, according to the claim 1, **characterized in that** the reactor used during the stage iii) is of a Lurgi type, a tubular reactor with the tubes packed with catalyst and cooled with boiling water, which is also obtained from the solar thermal power plant.

4. Industrial procedure to obtain lower alcohols from solar energy, according to the claim 1, **characterized in that** the catalytic reaction of the stage iv) occurs at a temperature of 420°C and at atmospheric pressure.
